Europäisches Patentamt

European Patent Office

Office européen des brevets

(11) Publication number: **0 138 946**
**B1**

# EUROPEAN PATENT SPECIFICATION

(45) Date of publication of patent specification: **06.12.89**

(21) Application number: **84901475.8**

(22) Date of filing: **05.03.84**

(86) International application number:
**PCT/US84/00338**

(87) International publication number:
**WO 84/04168 25.10.84 Gazette 84/25**

(51) Int. Cl.⁴: **G 01 N 33/574,**
**G 01 N 33/577**

(54) **Methods for monitoring the status of cancer in humans.**

(30) Priority: **18.04.83 PCt/us83/00579**
**16.09.83 US 532998**

(43) Date of publication of application:
**02.05.85 Bulletin 85/18**

(45) Publication of the grant of the patent:
**06.12.89 Bulletin 89/49**

(84) Designated Contracting States:
**FR**

(56) References cited:
**none**

(73) Proprietor: **SRI INTERNATIONAL**
**333 Ravenswood Avenue**
**Menlo Park, California 94025 (US)**

(72) Inventor: **VOLD, Barbara, Schneider**
**200 Waverley Street Apartment 5**
**Menlo Park, CA 94025 (US)**

(74) Representative: **Santarelli, Marc et al**
**Cabinet Rinuy et Santarelli 14, avenue de la**
**Grande Armée**
**F-75017 Paris (FR)**

Courier Press, Leamington Spa, England.

**Description**

This invention relates to a method of monitoring the status of cancer in human patients and is an extension of the technique disclosed in WO—A—83/03903 of the same applicant.

This prior application, which benefits from an earlier date but was only published on November 10, 1983, is only opposable to the present case on the level of novelty, as provided in articles 54(3) and (4) of the EPC.

In WO—A—83/03903 a method for determining whether a human patient has cancer is disclosed which comprises determining the amount of a specific cancer marker in the urine of the patient, and comparing said amount with the amounts associated with normal healthy humans, the specific cancer marker being N - [9 - (beta - D - ribofuranosyl)purin - 6 - ylcarbamoyl] - L - threonine, hereinafter referred to as $t^6A$.

According to the present invention, this method is extended to the monitoring of the status of the cancer by determining the amounts of $t^6A$ in the urine of the patient at different times and comparing the values obtained.

Accordingly, the present invention is directed to a method of monitoring the status of the cancer in human cancer patient, which comprises obtaining urine specimens from the patient at different times, determining the amounts of $t^6A$ in the specimens and comparing the values obtained.

The determination of the amounts of $t^6A$ may be made by a quantitative immunoassay in which a urine sample from the patient is incubated with an antibody against $t^6A$ and labelled $t^6A$ or by an enzyme-linked immunosorbent assay in which a $t^6A$-protein carrier conjugate bound to a solid phase is incubated in a competitive immunoassay with the urine sample and $t^6A$ antibody followed by incubation with an enzyme-labelled antibody against said $t^6A$ antibody and final determination of the enzyme label.

Preferably, the $t^6A$-antibody is a monoclonal $t^6A$-antibody and in particular such a monoclonal antibody obtained from hybridoma ATCC HB8351.

For use in such a method, there is also provided in accordance with this invention, an immunoassay kit comprising in association.

a) $t^6A$ conjugated to a carrier protein;

b) $t^6A$ antibody and

c) an enzyme-labelled antibody against said antibody or a functional equivalent thereof.

The invention is illutrated in the enclosed drawings, in which

Fig. 1 is a graph showing the results of the radioimmunoassay tests described in Example 2, infra,

Fig. 2 is a graph showing the results of the two ELISA tests described in Example 2, infra, and

Fig. 3 is a graph showing the results of the competition ELISA test described in Example 2, infra.

The method according to the invention may be used to monitor the status of any type of cellular neoplasm, including carcinomas, myelomas, and lymphomas. These methods are thus useful in diagnosis as in screening programs for early detection of the disease, in therapy as in evaluating the status of the disease after surgical radiation and/or chemotherapeutic treatment and in prognosis such as in detecting the possibility of recurrence or metastases. Examples of cancers that have been determined to be associated with elevated levels of modified nucleosides are leukemia, lung cancer, Burkitt's lymphoma, melanoma, ovarian cancer, metastic breast cancer, Hodgkin's disease, colon cancer, and bladder cancer.

The urine samples that are used in the invention methods may be collected randomly or over a given time period, conventionally 24 h. It has been reported that no significant variation in the levels of modified nucleoside occurs relative to the sample collection regimen. Fischbein, A. et al, *Cancer Res.* (1983) 43:2971—2974. One of the significant advantages of the invention is that the nucleoside does not have to be separated from the urine sample prior to being assayed. In the HPLC technique the nucleosides are separated by affinity chromatography from the sample before being assayed. The nucleosides also do not have to be derivatized as in the prior art serum assays. The amount of urine used in the invention methods will usually range between 1 to 100 µl depending upon the particular nucleoside and/or antibody involved.

In the method of the invention, and as opposed to the method of diagnosis of WO—A—83/03903, the patient's own levels of modified nucleoside in his/her urine serve as an internal control. Decreases signify a reduction in tumor burden such as might occur after the patient has been treated. Increases signify an increase in tumor burden indicating a recurrence or metastases of the cancer.

The amounts of these modified nucleosides in urine are determined by a quantitative immunoassay. Various types of quantitative immunoassays such as competitive immunoassays, direct immunoassays or indirect immunoassays may be used. The three most common quantitative immunoassays are radioimmunoassay, (both soluble phase and solid phase) quantitative immunofluorescence, and quantitative enzyme assays. Any of these may be used to practice the invention. These assays involve the formation of immune complexes that include a label and the detection of such complexes via the label. As used herein the term "label" is intended to include moieties that may be detected directly such as fluorochromes and radiolabels as well as moieties such as enzymes that must be reacted or derivatized to be detected. In competitive assays the sample is incubated with an antibody against the modified nucleoside and a known amount of a labelled modified nucleoside. Any modified nucleoside (unlabelled) in the sample competes with the labelled nucleoside for antibody. The resulting immune complexes are separated and the amount of labelled complex therein is determined. The amount of modified nucleoside is determined by comparison with the effect of standards. Direct immunoassays involve incubating the sample with a labelled antibody against the modified nucleoside and separating any immune complexes that form. The amount of label therein is determined and the amount of modified nucleoside in the sample is determined by comparison with the effect of standards. In an indirect immunoassay, such as an enzyme linked immunosorbent assay (ELISA), the antigen bound to a carrier protein such as an albumin is bound (immobilized) to a solid phase (e.g., a tube, bead or well surface), the first antibody is added, and a second enzyme-labelled antibody against the first antibody is added. Immobilized immune complexes are detected via reaction of the enzyme with an appropriate substrate.

The particular label that is used will depend on the type of quantitative immunoassay used. The assay should be such as to provide sensitivities down to at least 1 to 10 pmol of modified nucleoside. Examples of labels that may be used are radiolabels such as $^{32}P$, $^{125}I$, $^3H$, and $^{14}C$, fluorescent labels such as fluorescein and its derivatives, rhodamine and its derivatives, dansyl, and umbelliferone, chemiluminescers such as luciferia and 2,3-dihydrophthalazinediones, and enzymes, such as horseradish peroxidase, alkaline phosphatase, lysozyme, and glucose-6-phosphate dehydrogenase. The antibody or nucleoside, as the case may be, may be tagged with such labels by known methods. For instance, coupling agents such as aldehydes, carbodiimides, dimaleimide, imidates, succinimides and bis-diazotized benzadine may be used to tag the antibodies with fluorescent, chemi-luminescent, or enzyme labels.

The antibodies against the modified nucleosides that are used in these immunoassays may be polyclonal (heterologous) or monoclonal (homologous). Polyclonal antibodies may be made by immunizing host animals such as mice, rabbits, and sheep using a modified nucleoside-protein carrier conjugate as an immunogen. The immunization will typically involve repeated inoculations with the immunogen, typically at least two at about one week intervals. Such inoculation will raise an immune response against the immunogen and cause the inoculated host's immune system to produce antibodies against the modified nucleoside. Serum from the immunized host will usually be collected about three to ten days after the final booster. Immunoglobulins may be separated from the serum by ammonium sulfate precipitation, gel electrophoresis, dialysis, chromatography, or other conventional separation and purification techniques, if desired.

Monoclonal antibodies against the modified nucleosides may be made by the somatic cell hybridization techniques of Kohler and Milstein, *Nature* (1975) 265:495 using antibody-producing cells, e.g. spleen or lymphoid cells, from the immunized host animal, preferably a mouse, as one of the hybridization partners. The antibody-producing cells are hybridized (fused) with an appropriate cancer (myeloma) cell line using a fusogen such as polyethylene glycol having a Mw of 1,000 to 6,000 daltons. A myeloma cell line that is sensitive to a selective medium such as HAT medium (Littlefield, *Science* (1969) 145:709—710), fuses efficiently, and will support stable high level expression and secretion of antibody by its hybridization partner is used. While myeloma cells from any species such as those indicated above with respect to polyclonal antibodies may be used, murine and rat myeloma lines having these characteristics are available currently and are preferred. Examples of such lines are those derived from the original MOPC-21 and MPC-11 mouse tumors that are available from the Salk Institute Cell Distribution Center, PO Box 1809, San Diego, California 92112. A myeloma cell:antibody-producing cell ratio in the range of about 1:10 and about 10:1 will normally be used. The individual cell concentrations will typically be in the range of about $10^6$ to $10^8$, preferably $1 \times 10^7$ to $5 \times 10^7$, cells/ml fusion medium. Balanced salt solutions containing about 30% to 60% (w/v), preferably 35% to 50% (w/v) fusogen may be used as a fusion medium. After the fusion, the cells are washed with fusogen-free medium to remove fusogen. They are then seeded and cultivated in the selective medium to eliminate unhybridized parent cells and leave only hybrids that are resistant to the selective medium and possess the immortality of the myeloma parent. The cultivation will normally take about three to five weeks.

Surviving hybridomas may be examined for production of antibody against the modified nucleoside by immunoassay. Positive hybridoma clones may be subcloned by limiting dilution techniques and grown *in vitro* or *in vivo* by known techniques. The monoclonal anti-modified nucleoside antibody secreted by the subclones may be separated from the culture medium or ascites fluid when grown *in vivo* by known techniques such as ammonium sulfate precipitation, DEAE cellulose chromatography, or affinity chromatography. Further purification of the antibodies, if desired, may be achieved by ultracentrifugation and microfiltration.

Antibodies against the isotype of the antimodified nucleoside antibody may be used as the labelled second antibody in indirect assays such as the ELISA. For instance if the anti-modified nucleoside antibody is a murine IgG, anti-murine IgG serum from another mammalian species may be used. Such anti-isotype antibodies may be raised by conventional immunization techniques.

The incubations of the immunoassays will be carried out under conditions that permit reaction between the antibody and modified nucleoside. Temperature, pH and duration are the most important process conditions in the incubation. Temperatures in the range of about 5°C to 40°C, preferably about 37°C, will typically be used. The pH will normally be about 6 to 9, preferably about 7, and the binding reaction will usually reach equilibrium in about 10 min to 2 days, depending upon the particular antibodies and nucleosides involved. Antibody will be used in limiting amounts in the competitive assays. Immune complexes may be separated from the incubation mixture by centrifugation or other conventional techniques.

Separation may not be necessary in assays such as the homogeneous enzyme immunoassay (EMIT). Conventional buffer media may be used for any washing steps involved in the assays.

The label detection means used in the immunoassay will depend upon the particular label involved. For instance, radiolabels may be read with scintillation counters, fluorescent labels with fluorescent microscopes (fluorometers) and enzyme labels with colorimeters that detect the magnitude of color change caused by the reaction between the enzyme and the substrate or spectrophotometers (e.g., microtiter plate readers).

The basic ingredients of the test kit for carrying out the $t^6A$ radioimmunoassay of the invention are labelled $t^6A$ and monoclonal antibody against $t^6A$. These ingredients will typically be in solution in an appropriate aqueous solvent and contained in suitable dispensers. Solid phase kits will contain a solid phase (tube or bead) and, optionally, a coupling agent such as an aldehyde or carbodiimide to bind the antibody to the solid phase. ELISA kits will contain $t^6A$-carrier protein conjugate, anti-$t^6A$ monoclonal antibody, enzyme-labelled antibody against the monoclonal antibody, and a suitable substrate. The kits may also contain a suitable buffer for dilution and washes, carrier protein, unlabelled $t^6A$ for preparing standards, a post-coating preparation to reduce nonspecific bonding to solid phase, containers in which the reagents may be used, and directions for performing the assay. The components of the kit may be packaged in a conventional manner.

As indicated above and as illustrated by the examples, *infra*, in which rabbit anti-$t^6A$ serum and murine monoclonal anti-$t^6A$ antibody are used in the invention, the species of the antibody is not critical. Likewise, as evidenced by the use of rabbit antisera in the invention method (Example 1, infra) the class (subclass) of antibody does not appear to be critical. In this regard although only a single anti-$t^6A$ monoclonal antibody is described in the examples, it is intended that the invention encompass all anti-$t^6A$ monoclonal antibodies, regardless of species or class, that are functionally equivalent to the described monoclonal antibody. Functionally equivalent monoclonal antibodies are those that crossblock (react with the same determinant) the described monoclonal antibody. In this regard the subclasses of IgG differ from one another in their constant region. However, an IgG against a specific antibody will have a functionally equivalent variable region regardless of its subclass. Accordingly, although the exemplified monoclonal antibody is an IgG of a particular subclass, functional monoclonal antibodies of other IgG subclasses or other classes (IgM, IgA, etc.) are intended to be within the scope of the invention.

The following examples further illustrate the invention methods. These examples are not intended to limit the invention in any manner.

Example 1
Urine Samples

24 h urine samples were obtained from patients with various advanced malignancies, previously untreated with chemotherapy, and normal volunteers at the Cancer Center of the University of New Mexico. The urines were collected from each individual in plastic containers for 24 h and kept under refrigeration until the end of collection. The urine volume was then measured and an aliquot of urine was kept frozen until the day of analysis. The amount of creatinine for each sample was determined by the colorimetric method described in the Sigma Technical Bulletin No. 555 1—12, 1977.

Antibody Preparation

Immunogen was prepared by conjugating $t^6A$ to bovine serum albumin (BSA) using the method of Erlanger and Beiser, *PNAS* (1964) *52*:68—74. Antibodies were made in rabbits, a total of 5 mg immunogen (mixed with complete Freund's adjuvant) per rabbit was introduced in three injections into the hind legs on days 0, 14, and 21. The immunized rabbits were bled on day 28. Serum was precipitated twice at 0°C with 50% saturated ammonium sulfate (SAS), pH 7.0, and then dialyzed against 0.14 M NaCl, 10 mM sodium phosphate, pH 7.0 (PBS).

Tritiated Antigen

Tritiated $t^6A$ was prepared by catalytic (Pt) exchange with tritiated water. $[^3H]t^6A$, $3.1\times10^{10}$ Bq/mmol (0.84 Ci/mmol), was 88% pure by 2D thin layer chromatography analysis using the solvent system described by Rogg, et al., *Nucleic Acids Res.* (1976) 3:285—295, and 6.9 pmol was used per assay.

Radioimmunoassay

The saturated ammonium sulfate radioimmunoassay (SAS-RIA) for $t^6A$ described by Vold, B. S., *Nucleic Acids Res.* (1979)7:193—204 was used. Assays were done in 12×75 mm polypropylene tubes in a total reaction volume of 300 µl with 75 mM NaCl, 0.1 M boric acid, 25 mM sodium tetraborate, pH 8.3. Normal rabbit IgG was used to adjust the total protein concentration of each assay to 600 µg. The buffer, antibody, carrier protein, $[^3H]t^6A$, and untreated urine were mixed well, incubated at 37°C for 1 h, then cooled on ice for 5 min. The amount of urine used was chosen such that normal urine would give inhibition values between 10% and 40%. Thus, 2 µl of urine was used in each assay. An equal volume of ice-cold SAS was added to each tube, mixed thoroughly, and incubated at 0°C for 30 min. The tubes were then centrifuged at 9,400×g for 15 min at 4°C, and the supernatant removed by aspiration through a capillary pipette. The pellets were resuspended in 200 µl of 50% SAS in borate saline buffer, mixed, centrifuged, and drained as before. The washed pellets were then suspended in 200 µl of 1× borate saline buffer, mixed, and 75 µl added to 5 ml of Aquasol® scintillation fluid. Radioactivity was then measured in a scintillation counter. Under the conditions used for competitive inhibition, cpm bound on the filter without inhibitor and with (or without) antiserum was 1133 (66) for $t^6A$. When urine was added as a source of competitive inhibitor and the same assay repeated on four different occasions, standard deviation was ±3.7%.

The concentration of inhibitor present in each urine sample was interpolated from a graph of % trace binding for the antibody when the cognate modified nucleoside had been used as competitive inhibitor. These concentrations were then expressed in mg excreted per 24 h period and normalized to the creatinine level in each sample expressed as nmol nucleoside/µmol creatinine. Speer, et al, *Cancer* (1979) 44:20—2123.

Urine samples from 8 normal patients, 4 Hodgkins patients, 4 non-Hodgkin's lymphoma patients, 5 lung cancer patients (small cell, adenocarcinoma, squamous cell and large cell) and 8 "other" cancer (breast, head and neck, bowel) patients were tested. None of the patients had been treated previously by chemotherapy. Assay results for the cancer patients were compared by group with the assay results from the normal patients. Significance of variation in the levels of $t^6A$ in the samples was established using a t test comparing each cancer group to the normal group. A P value less than 0.05 was considered a significant variation. The results of these comparisons were:

Significance of variation (P)

| Lymphomas | | Solid tumors | |
|---|---|---|---|
| Hodgkins | Non-Hodgkins | Lung | Other |
| not significant | P<0.05 | P<0.001 | P<0.02 |

As these data indicate elevated levels of $t^6A$ in urine is a clear marker for all the cancers tested except perhaps Hodgkins disease. The lack of significant variation in the Hodgkins patients may be attributable to the small number of patients involved.

Example 2
Monoclonal Antibody Preparation

A Balb/C mouse was immunzied with the $t^6A$-BSA conjugate of Example 1 (100 µg) in complete Freund's adjuvant intraperitoneally at day 0. At day 7 the mouse was boosted with 100 µg of the conjugate in PBS intravenously. The mouse spleen was removed at day 10. Spleen cells were fused with P3/63/Ag 8.653 murine myeloma cells according to Kennet's fusion protocol (Kennet, et al, (1979) *Cell Fusion*. In Methods in Enzymology, Academic Press, New York pp. 345—357). The fusogen was polyethylene glycol (1540 mw) at 38% w/v.

Media from wells containing surviving cells were tested for anti-$t^6A$ antibody by radioimmunoassay Cells from one of the positive wells (# G8) were subcloned by limiting dilution into wells containing normal mouse spleen cells as feeders. Subclones were tested for antibody production by radioimmunoassay and one, identified as G8-3, was chosen as a source of monoclonal anti-$t^6A$ antibody.

Cells from clone G8-3 were then frozen, reestablished in culture, and subcloned a second time *in vitro* according to the same procedure as the first sub-cloning. Two sub clones from the second sub-cloning were retained (#9D4 and #5C4). Cells from line #9D4 were selected to be the established cell line.

The screening of the clones was done using a solid phase assay. A conjugate of $t^6A$ to human serum albumin was made and used to coat wells of a microtiter plate. Culture fluid was then added followed by a second antibody which was labelled with $^{125}I$ and which recognizes mouse IgG. A sample of line #9D4 was deposited in the American Type Culture Collection 12301 Parklawn Drive, Rockville, Maryland 20852, USA on August 23, 1983. This sample was assigned ATCC number HB 8351.

Protein from the culture fluid of #9D4 was isolated by means of an affinity column chromatography step using Protein A-Sepharose® CL-4B. The bound fraction from this column was tested in various quantitative immunoassays for $t^6A$ as described below.

Radioimmunoassay

The SAS-RIA referred to in Example 1 was used. Reaction volume was 300 µl, and the total protein content was normalized using carrier normal rabbit immunoglobulins—typically 300 µg of total protein/assay. Reactions containing 80 µg of the above described fraction, 15 pmol $[^3H]t^6A$ varying concentrations of unlabelled $t^6A$, and carrier protein were incubated for 1 h at 37°C in borate-saline buffer: 75 mM NaCl, 0.1 M boric acid, 25 mM sodium tetraborate, pH 8.3. After the reactions were cooled on ice, an equal volume of cold SAS was added. The solution was mixed thoroughly, and the incubation was continued for 30 min at 0°C. At the end of the 30-min incubation, the suspensions were centrifuged for 15 min at 4°C and 9,400×$g$ and the supernatant fluid was removed by aspiration. The pellet was resuspended in 500 µl of ice-cold 50% SAS in borate-saline buffer, mixed, recentrifuged, and drained as before. The washed pellets were then resuspended in 500 µl of borate-saline buffer and transferred to 5 ml of Aquasol

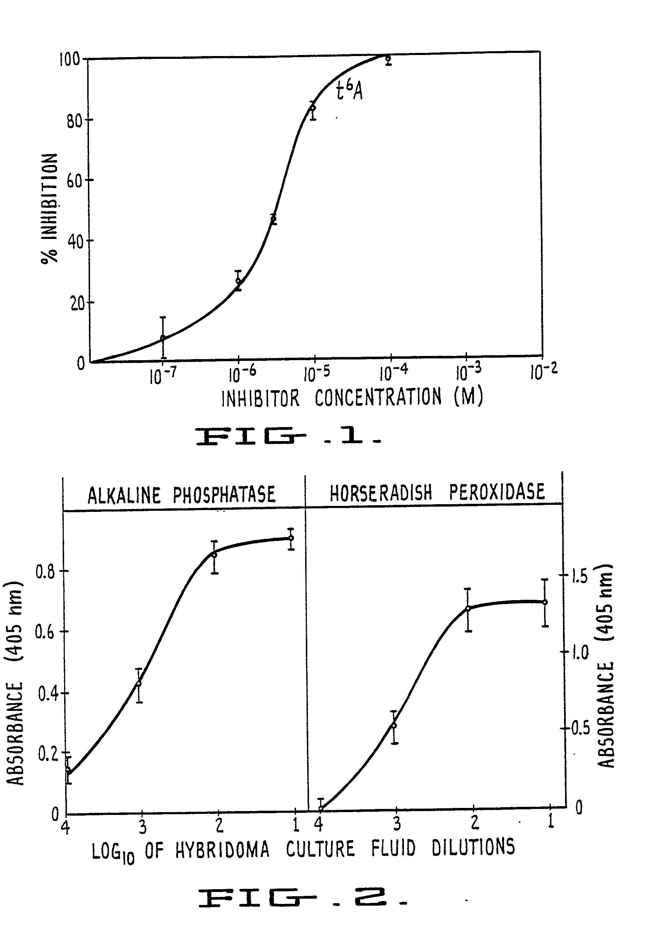

scintillation fluid. Radioactivity was measured in a scintillation spectrophotometer. The results are shown in Fig. 1.

ELISAs

Two ELISA techniques were tested: one using alkaline phosphatase, the other horseradish peroxidase (HRP). In each technique, microtiter plates were washed once with tap water and once with distilled water. Then each well was filled with 50 µl of 10 µg/ml t⁶A conjugated to human serum albumin (HSA-t⁶A) in 1 M NaCl and 0.02 M sodium phosphate (pH 7.0), and left overnight at 4°C. Each well was emptied and washed (3×100 µl) with 1% HSA in PBS. Wells were then filled with a solution without antibody or various dilutions of hybridoma culture fluid in PBS.

After incubation at room temperature for 2 to 3 h, wells were emptied and washed (3×100 µl) with 1% HSA in PBS. Then each well was incubated with 50 µl of a dilution of enzyme-anti-mouse IgG conjugate. Each type of reaction was then treated differently, as described below.

Alkaline phosphatase

To each well was added 50 µl of a 1/500 dilution of goat anti-mouse IgG conjugated to alkaline phosphatase. After standing at room temperature for 2 to 3 h, the wells were emptied, washed (3×100 µl) with 10% diethanolamine (pH 9.8), and filled with 100 µl of 1 mg/ml p-nitrophenylphosphate in 10% diethanolamine (pH 9.8). The enzyme-substrate reaction was incubated 30 min at room temperature and stopped with 100 µl of 3N NaOH. Absorbance at 405 nm was read in an Automated Microtiter Plate Reader (Micro ELISA Autoreader® MR 580, Dynatech).

Horseradish peroxidase

To each well was added 50 µl of a 1/500 dilution of goat anti-mouse IgG conjugated to horseradish peroxidase. After standing at room temperature for 2 to 3 h, the wells were emptied and washed once with water and twice with 0.05% Triton® X-100 surfactant in PBS. To each well was added 100 µl of the following solution: 0.1 ml of 15 mg 2,2′ - azino - bis - (3 - ethylbenzthiazoline sulfonic acid) (ABTS) per ml of $H_2O$, 0.33 ml of 0.3% $H_2O_2$, and 10 ml of citrate buffer (0.105 g of citric acid in 10 ml of $H_2O$ adjusted to pH 4.0 using 5 M NaOH). After 30 min, absorbance at 405 nm was read in an Automated Microtiter Plate Reader.

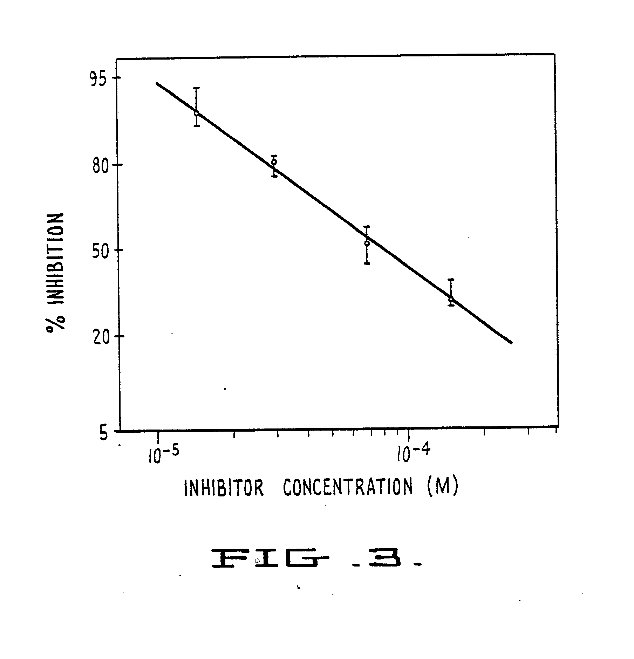

Fig. 2 shows the results of these ELISAs.

A standard curve using a t⁶A standard can be prepared for these ELISA techniques using a competitive inhibition procedure in which free t⁶A is added to the wells in which the t⁶A-HSA has been absorbed. A standard curve with the alkaline phosphatase ELISA is shown in Fig. 3. Figure 3 demonstrates that 50% inhibition of the ELISA was achieved at a concentration of t⁶A of $8.4×10^{-5}$ M. Using the RIA with the monoclonal antibody gave 50% inhibition at $3.2×10^{-6}$ M. The concentration of t⁶A in human urine is approximately $15×10^{-6}$ M.

**Claims**

1. A method for monitoring the status of cancer in a human cancer patient characterized by
   (a) obtaining urine specimens from the patient at differnt times;
   (b) determining the amounts of N - [9 - (beta - D - ribofuranosyl)purin - 6 - ylcarbamoyl] - L - threonine (t⁶A) in the specimens; and
   (c) comparing the amounts, an increase in amount being an indication of increased tumor burden and a decrease in amount being an indication of decreased tumor burden.

2. The method of claim 1 wherein the cancer is a non-Hodgkins lymphoma or a solid tumor.

3. The method of claim 1 or 2 wherein the determinations of step (b) are made by a quantitative immunoassay in which a urine sample from the patient is incubated with an antibody against N - [9 - (beta - D - ribofuranosyl)purin - 6 - ylcarbamoyl] - L - threonine and labelled N - [9 - (beta - D - ribofuranosyl)purin - 6 - ylcarbamoyl] - L - threonine.

4. The method of claim 3 wherein the antibody is a monoclonal antibody in particular a monoclonal antibody from hybridoma ATCC HB 8351.

5. The method of claim 1 or 2 wherein the determinations of stetp (b) are made by an enzyme-linked immunosorbent assay in which a t⁶A-protein carrier conjugate bound to a solid phase is incubated in a competitive immunoassay with the urine sample and t⁶A antibody followed by incubation with an enzyme labelled antibody against said t⁶A antibody, and the final determination of the enzyme label.

6. The method of claim 5 wherein the t⁶A antibody is a monoclonal antibody, in particular a monoclonal antibody from hybridoma ATCC HB 8351.

7. An immunoassay kit for determining the amount of N - [9 - (beta - D - ribofuranosyl)purin - 6 - ylcarbamoyl] - L - threonine (t⁶A) in a sample according to the method of any one of claims 1 to 4 characterized in that the kit comprises in association:
   (a) t⁶A conjugated to a carrier protein;
   (b) t⁶A antibody; and
   (c) an enzyme labelled antibody against said t⁶A antibody.

8. A kit according to claim 7 wherein said t⁶A antibody is a monoclonal antibody.

9. A kit according to claim 8 wherein said t⁶A antibody is a monoclonal antibody from hybridoma ATCC HB 8351.

**Patentansprüche**

1. Verfahren zur Krebsüberwachung bei einem Krebspatienten, gekennzeichnet durch
a) Entnahme von Urinproben von dem Patienten zu verschiedenen Zeiten,
b) Bestimmung der Mengen von N⁻-[9-(beta-D-Ribofuranosyl)purin-6-yl-carbamoyl]-L-Threonin (t⁶A) in den Proben, und
c) Vergleich der Mengen, wobei eine Mengenzunahme ein Zeichen für eine größer gewordene Tumorlast und eine Mengenabnahme ein Zeichen für eine kleiner gewordene Tumorlast ist.

2. Verfahren nach Anspruch 1, bei dem der Krebs ein nicht-Hodgkin-Lymphom oder ein fester Tumor ist.

3. Verfahren nach Anspruch 1 oder 2, in dem die Bestimmungen von Schritt (b) mittels eines quantitativen Immuntests durchgeführt werden, bei dem eine Urinprobe von dem Patienten mit einem gegen N-[9-(beta-D-Ribofuranosyl)purin-6-yl-carbamoyl]-L-Threonin und markiertes N-[9-(beta-D-Ribofuranosyl)purin-6-yl-carbamoyl]-L-Threonin gerichteten Antikörper inkubiert wird.

4. Verfahren nach Anspruch 3, in dem der Antikörper ein monoclonaler Antikörper, insbesondere ein monoclonaler Antikörper der Hybridomazellen ATCC HB 8351 ist.

5. Verfahren nach Anspruch 1 oder 2, in dem die Bestimmungen von Schritt (b) mittels eines enzymverbundenen Immunadsorptionstests durchgeführt werden, bei dem ein an eine Festphase gebundenes t⁶A-Protein-Träger-Konjugat in einem kompetitiven Immuntest mit der Urinprobe und dem t⁶A Antikörper inkubiert wird und sodann eine Inkubation mit einem gegen den t⁶A Antikörper gerichteten enzymmarkierten Antikörper und die End-Bestimmung der Enzymmarkierung erfolgt.

6. Verfahren nach Anspruch 5, in dem der t⁶A Antikörper ein monoclonaler Antikörper, insbesondere ein monoclonaler Antikörper der Hybridomazellen ATCC HB 8351 ist.

7. Immuntest-Kit zur Bestimmung der Menge von N-[9-(beta-D-Ribofuranosyl)purin-6-yl-carbamoyl]-L-Threonin (t⁶A) in einer Probe gemäß dem Verfahren nach irgendeinem der Ansprüche 1 bis 4, dadurch gekennzeichnet, daß der Kit umfaßt:
a) t⁶A konjugiert mit einem Trägerprotein,
b) t⁶A Antikörper, und
c) enzymmarkierten, gegen den t⁶A Antikörper gerichteten Antikörper.

8. Kit nach Anspruch 7, in dem der t⁶A Antikörper ein monoclonaler Antikörper ist.

9. Kit nach Anspruch 8, in dem der t⁶A Antikörper ein monoclonaler Antikörper der Hybridomazellen ATCC HB 8351 ist.

**Revendications**

1. Procédé pour contrôler l'état du cancer chez un sujet humain atteint du cancer, caractérisé en ce qu'il consiste
(a) à prélever des échantillons d'urine chez le patient à des temps différents;
(b) à déterminer les quantités de N-[9-(bêta-D-ribofurannosyl)purine-6-ylcarbamoyl]-L-thréonine (t⁶A) dans les échantillons; et
(c) à comparer les quantités, un accroissement de quantité constituant une indication d'une charge tumorale accrue et une diminution de quantité constituant une indication d'une charge tumorale réduite.

2. Procédé suivant la revendication 1, dans lequel le cancer est un lymphome de type non-Hodgkinien ou une tumeur solide.

3. Procédé suivant la revendication 1 ou 2, dans lequel les déterminations de l'étape (b) sont effectuées par un immuno-essai quantitatif dans lequel un échantillon d'urine provenant du patient est incubé avec un anticorps contre la N-[9-(bêta-D-ribofurannosyl)purine-6-ylcarbamoyl]-L-thréonine et de la N-[9-(bêta-D-ribofurannosyl)purine-6-ylcarbamoyl]-L-thréonine marquée.

4. Procédé suivant la revendication 3, dans lequel l'anticorps est un anticorps monoclonal, en particulier un anticorps monoclonal provenant de l'hybridome ATCC HB 8351.

5. Procédé suivant la revendication 1 ou 2, dans lequel les déterminations de l'étape (b) sont effectuées par analyse avec un immuno-sorbant lié à un enzyme, dans laquelle un conjugué t⁶A-support protéique lié à une phase solide est incubé dans un immuno-essai compétitif avec l'échantillon d'urine et un anticorps contre la t⁶A, puis est incubé avec un anticorps, marqué avec un enzyme, contre ledit anticorps contre la t⁶A, et par un dosage final du marqueur enzymatique.

6. Procédé suivant la revendication 5, dans lequel l'anticorps contre la t⁶A est un anticorps monoclonal, en particulier un anticorps monoclonal provenant de l'hybridome ATCC HB 8351.

7. Kit pour immuno-essai, destiné à déterminer la quantité de N-[9-(bêta-D-ribo-furannosyl)purine-6-ylcarbamoyl]-L-thréonine (t⁶A) dans un échantillon conformément au procédé suivant l'une quelconque des revendications 1 à 4, caractérisé en ce qu'il comprend, en association:

(a) de la t$^6$A conjuguée à une protéine servant de support;

(b) un anticorps contre la t$^6$A; et

(c) un anticorps, marqué avec un enzyme, contre ledit anticorps contre la t$^6$A.

8. Kit suivant la revendication 7, dans lequel l'anticorps contre la t$^6$A est un anticorps monoclonal.

9. Kit suivant la revendication 8, dans lequel l'anticorps contre la t$^6$A est un anticorps monoclonal provenant de l'hybridome ATCC HB 8351.

EP 0 138 946 B1

FIG.1.

FIG.2.

FIG .3.